# EUROPEAN PATENT SPECIFICATION

(11) **EP 0 787 227 B1**
(45) Date of publication and mention of the grant of the patent: **09.06.1999**
(21) Application number: 96926236.9
(22) Date of filing: 01.08.1996
(51) Int. Cl.: D04H 3/05

(54) **PROCESS AND APPARATUS FOR MAKING COMPOSITE SHEET**
VERFAHREN UND VORRICHTUNG ZUR HERSTELLUNG EINER VERBUNDFOLIE
PROCEDE ET APPAREIL POUR LA FABRICATION D'UNE FEUILLE COMPOSITE

(30) Priority: 03.08.1995 US 510994
(43) Date of publication of application: 06.08.1997
(73) Proprietor: E.I. DU PONT DE NEMOURS AND COMPANY, Wilmington Delaware 19898 (US)
(72) Inventor: WARD, Robert, Roch, Hockessin, DE 19707-1208 (US); BENIN, Joshua, Newark, DE 19711-2308 (US); HAMILTON, Cathy, Jane, Wilmington, DE 19807-2427 (US); TALO, Leslie, C., Neenah, WI 54956 (US)
(74) Representative: Hughes, Andrea Michelle
(86) International application number: US9612646
(87) International publication number: WO9706299

(56) References cited:
- US-A- 2 812 797
- US-A- 2 962 080
- US-A- 3 607 565
- US-A- 3 878 591
- US-A- 4 080 232
- US-A- 5 000 806

## Description

This invention relates to a process and apparatus for making a composite sheet material wherein a strand is attached transversely to a moving sheet. More particularly, the invention concerns a high speed process and apparatus in which a strand, carried by a reciprocating strand traverse guide that is driven by a cylindrical cam, is looped around pins projecting from a pair of pin conveyors which then transfer the strand from the pins to the surface of the moving sheet. The invention is especially useful for high speed manufacture of elastic composite sheet material that can be attached as elastic components to disposable diapers, adult incontinence articles and other garments.

Processes and apparatus are known in which a continuous filament or strand is laid across and attached to the surface of moving sheet. For example, Estee et al, United States Patent 2,812,797, discloses an apparatus for making a laminated fabric. In the apparatus, a sheet is continuously advanced between two endless belts that travel in unison and in the same direction as the sheet. One belt is located at each edge of the sheet and each belt has a series of pins projecting from its surface. A swinging frame traverses a plurality of continuous filaments back and forth across the sheet. At each reversal of the traverse, each filament from the frame is looped around a corresponding pin of the belt. Adhesive is applied to the sheet and the filaments. Then, the filaments are disengaged from the pins and adhered to the sheet surface to form an assembly of filaments and sheet. A cover sheet is placed atop the assembly and passed with the filament/sheet assembly between calender rolls to produce the laminated fabric. In another method, disclosed by Hirsch, U. S. Patent 2,962,080, an endless chain mechanism equipped with spaced apart pins, or a swinging lever mechanism equipped with a thread guide, traverses a thread across a sheet that is moving perpendicularly to the path of the traverse. Outside each edge of the sheet is a moving endless chain, equipped with a plurality of spaced apart pins. The thread from the traversing mechanism, at one end of each traverse path, deposits the thread around a pin on the chain at one edge of the sheet and then at the other end of the traverse path deposits thread on a pin of the chain on the other edge of the sheet. Although this method is suitable for use with non-elastic thread, the method is slow and unsuited for use with elastic thread.

Other methods for traversing filaments or strands across a moving surface to form reinforced sheets, scrims or nets also are known. For example, a reciprocating conveyor is disclosed by Friedrich, U. S. Patent 4,080,232, for laying threads on the outer surface of a rotating cylinder that has barbs projecting from both edges of the cylindrical surface to restrain the threads and form an open mesh net-like structure. Kelly et al, U. S. Patent 4,600,468, also discloses a method for laying out biased arrays of parallel yarns on conveyors equipped with needles to restrain the yarns and adhesively bonding the arrays together to form a bias-laid nonwoven fabric. A rotating arm is disclosed by Persson et al, U.S. Patent 5,221,390, for positioning conventional or elastic threads on "dogged elements" (e.g., pins or studs) projecting from a pair of belts located at the edges of a conveyor path. Merkatoris et al, U.S. Patent 5,000,806, discloses a method and apparatus for applying elastic strand to a disposable diaper by means of canted, spindle-equipped wheels for engaging the elastic strand, moving the strand into a sinuous configuration, stretching the strand and thereafter applying the strand transversely to an advancing component of the diaper.

Each of the above-described methods and apparatuses are rather complex and do not attain high speeds which are desired for efficient and economical attachment of strand in a transverse direction to a moving substrate. Further, some of these methods are not suited for handling elastic strands. Accordingly, an object of this invention is to provide a process and apparatus that will rapidly and economically attach elastic strand transversely to a moving sheet. Another object is to provide a garment having an elastic component made by the process and apparatus of the present invention.

US 3,607,565 and US 3,878,591 also disclose devices for forming a non-woven material in which a transverse thread feeder or shuttle moves to and from a row of pins of one side of a conveyor to a row on the opposite side.

Though not concerned with the problems associated with attaching strand to moving sheets, various mechanisms for traversing strand across a path are known. For example, Altice et al, U.S. Patent 3,086,722, and Akers et al, U.S. Patent 3,675,863, disclose yarn traversing guides driven by rotating cylindrical cams for high speed winding of yarn packages, such as bobbins, cakes and the like.

According to one aspect, the present invention provides a process for making a composite sheet material, wherein the process comprises
advancing in a longitudinal direction a sheet substrate, a first conveyor and second conveyor, the sheet substrate having two lateral edges and an upper and a lower surface and each conveyor having a plurality of spaced apart pins extending therefrom in a direction generally perpendicular to the movement of the conveyors, the conveyors being spaced apart and operating in synchronization,
supplying a strand to a device that reciprocates the strand to and fro across a traverse path, the traverse path being generally transverse to the longitudinal direction of the advancing sheet substrate,
advancing the conveyors to move the pins through a path that intersects the traverse path of the strand, thereby causing the strand to repetitively loop alternately, at each end of the traverse path, around a pin on the first conveyor and then around a pin on the second conveyor to form a strand array having edges carried by the pins,
applying an adhesive to the strand array or to the advancing sheet substrate or to both the strand array and the advancing sheet substrate,
transferring the strand array from the pins to the upper surface of the advancing sheet substrate to form a strand-sheet assembly, and forwarding the resultant composite sheet material to a windup or to further processing steps, characterised in that the process further comprises:
feeding the strand to a strand traverse guide driven by a rotating cylindrical cam,
moving the pins through an arcuate path that intersects the traverse path of the strand,
advancing the sheet substrate on a moving sheet support, and
restraining each edge of the strand array in position on the sheet substrate from a location after the point where the strand array is transferred from the pins to the advancing sheet substrate to a location beyond the point where the applied adhesive has become set.

Typically, the traverse reciprocating traverse guide has a stroke (i.e., one-half of a to-and-fro cycle) that is at least 5 centimetres long preferably at least 10 cm. Preferably, the strand is an elastic strand, most preferably a spandex. The elastic strand is elongated in the range of 10 to 400% beyond its original relaxed length while being supplied to the strand traverse guide and/or while being restrained in the strand array.

According to a second aspect, there is provided an apparatus for making a composite sheet material in which a strand supplied through a reciprocating guide is looped around pins of a pair of spaced-apart pin conveyors, the pin conveyors transfer the strand transversely to a sheet substrate advancing in a longitudinal direction and the strand is adhered to the sheet substrate, characterised in that:
the reciprocating guide is a lightweight strand guide connected to a cylindrical cam, the strand guide having a slotted tip for receiving the strand and a follower portion for fitting a groove in the cylindrical cam, so that when the cam is rotated the strand guide is driven to and fro along a traverse path having a first end and a second end;
the pin conveyors each have a plurality of evenly spaced-apart pins projecting from the surface of the conveyor, the first conveyor being positioned near the first end of the traverse path and the second conveyor being positioned near the second end of the traverse path, and the space between the conveyors being less than the distance between the first and second ends of the traverse path;
means for moving the pins of the conveyor through an arcuate path that intersects the traverse path, so that during operation strand carried by the reciprocating guide, near each end of each traverse, is looped around a pin of the pin conveyor;
a surface for supporting and moving the sheet substrate in the longitudinal direction into a position for transferring the strand from the pins to the sheet substrate, the transfer position being located between the pin conveyors and at a nip formed by a restraining means and the support surface;
means for synchronizing the relative speeds of the reciprocating traverse guide and the pin conveyors to assure the looping of strand on a pin of the conveyor at each end of the strand path; and
means for controlling the movement of the restraining means and the support surface at equal speeds.

According to a third aspect, there is provided a garment having installed therein an elastic component made according to the process of the invention.

The resultant composite sheet material when formed with non-elastic strand is reinforced in the transverse direction of the composite sheet material. Composite sheet that was formed with elastic strand is elastic in the transverse direction. Such an elasticized composite sheet material can be cut into tapes or swatches of suitable dimensions and fed directly to the elastic swatch applicator of a garment-making machine to form the desired elastic component in the garment.

Preferred embodiments of the invention will now be described, by way of example only, with reference to the accompanying drawings.
Fig. 1 is a side view schematic diagram of an apparatus for making composite sheet material according to the invention showing relative locations of housing **30** which holds a cylindrical cam and a strand traverse guide, pin-conveyor wheel **40** and sheet support drum **50**;
Fig. 2 is a partial top view of a portion of the apparatus of Fig. 1 showing strand **10** looped around the pins of **42**, **42'** of pin-conveyor wheels **40**, **40'**, and forming a strand array **16** on support drum **50**, the array being transferred atop moving sheet **12** and held in place on the drum surface by restraining belts **52**, **52'**;
Fig. 3 is an isometric view illustrating the operation of the portion of the equipment of Fig 1 that is located downstream of traverse guide **36**;
Fig. 4 is a side view of another suitable arrangement of housing **30**, pin-conveyor wheel **40**, sheet support drum **50** and restraining belt **52**;
Fig 5 is a side view of still another arrangement of housing **30**, pin-conveyor wheel **40**, sheet support drum **50** and restraining belt **52** for an apparatus of the invention for making composite sheet material **18** with a cover sheet **14**;
Figs. 6 and 7 are each an enlarged side view of an area enclosed by dashed lines in Fig. 1;
Fig. 8 is a partial cut-away front view of housing **30** showing cylindrical cam **32**, strand guide **36**, positioned between upper and lower traverse guide rails **34** and **35** respectively;
Fig. 9 is a front view of cylindrical cam **32** having groove **33** into which the cam follower portion of the strand guide is fitted and slides;
Fig. 10 a, b and c illustrate planar developments ofthree profiles of the groove **33** of cylindrical cam **32**;
Fig. 11a and 11b each depict in a detailed side-view cross-section, similar to that of Fig. 6, two geometries for guide rails **34** and 35;
Fig. 12 is a top view detailed cross-section of cam follower **38** in groove **33** of cylindrical cam **32** and of slotted tip **37** of strand traverse guide **36**;
Fig. 13 depicts another embodiment of the slotted tip **37** of strand guide **36**;
Fig. 14a and 14b are respectively a front view and a side view of pin-conveyor wheel **40**;
Fig. 15a and 15b illustrate in detailed side view two embodiments of pins **42** mounted on a pin-conveyor wheel **40**;
Fig. 16a and 16b respectively illustrate in top view two embodiments of pin-conveyor wheels **40** and **40'** canted inwardly or outwardly in relation to sheet **12** to be advanced on sheet support drum **50**; and
Fig. 17a, 17b, 17c, 17d and 17e each illustrate a different strand array **16** of strand **10** applied to advancing sheet **12**.

Preferred embodiments of the process and apparatus for making composite sheets in accordance with the invention will now be described with reference to the accompanying Figures.

The apparatus comprises three main components; namely, a cam-driven guide housing **30**, pin conveyors **40**, **40'** and a sheet forwarding and supporting unit **50**. Housing **30** contains cylindrical cam **32** that drives strand traverse guide **36** to cause strand **10** to be looped around pins **42**, **42'** of a pair of pin conveyors **40**, **40'** to form a strand array **16**. A sheet **12** is advanced on sheet support unit **50** and assembled with strand array **16** and optionally with a cover sheet **14** to form composite sheet material **18**, which then can be wound up or forwarded to further processing operations.

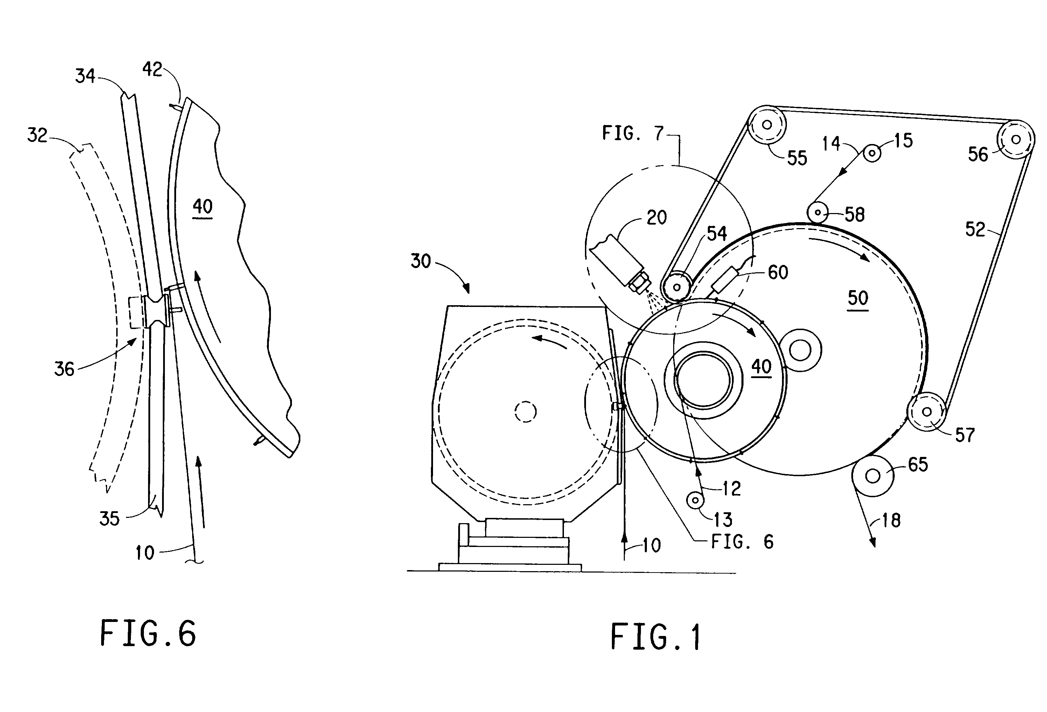

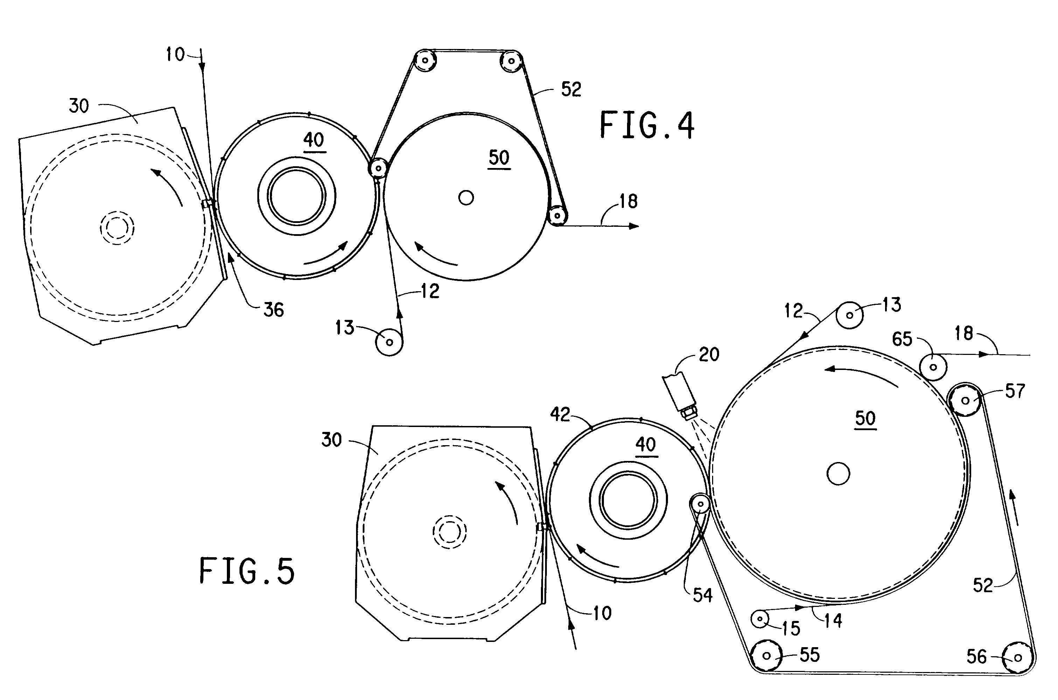

In accordance with the process of the invention, strand **10** is supplied to strand traverse guide **36** from below the guide as shown in Figs. 1 and 5, or from above the guide as shown in Fig. 4. When spandex is employed as the strand to be incorporated into the composite sheet material, a simple feed path is preferred for the strand from the supply to the traverse guide with as few idler rolls as practicable. One idler roll may suffice to feed the strand from a supply package (e.g., a bobbin or a cake) to the traverse guide. Such a feed path minimizes the amount of friction and tension to which the strand is subjected and permits smoother, more uniform feeding of strand to the apparatus. Typically, the spandex is supplied to the apparatus with an extension of 10 to 400% beyond the relaxed length of the spandex.

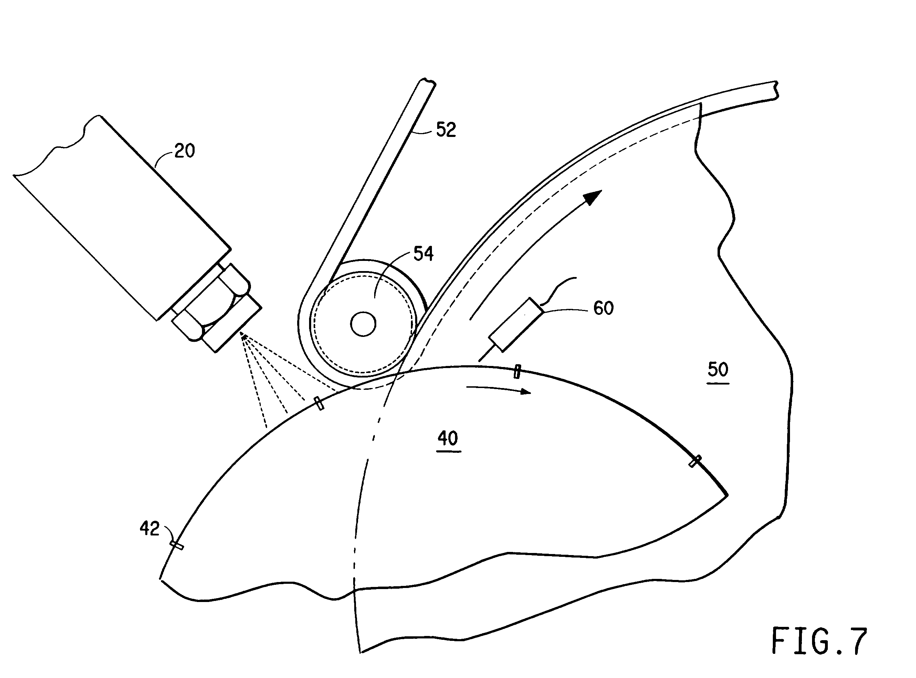

As illustrated in Figs. 6, 8, 11a, 11b and 12, strand traverse guide **36** has cam follower portion **38** that is mounted in groove **33** of rotating cylindrical cam **32**. Strand guide **36** rides between upper and lower rails **34** and **35**. As cylindrical cam **32** is rotated, strand traverse guide **36** traverses a path that is perpendicular to and intersected by the paths of pins **42**, **42'** carried by two spaced-apart pin conveyors. In the figures, the pin conveyors are illustrated as pin-conveyor wheels **40**, **40'**. Each wheel has a plurality of pins **42**, or **42'** projecting from its circumference. As wheels **40**, **40'** are rotated, strand **10** is looped by strand traverse guide **36** around each pin, first on a pin of one pin-conveyor wheel and then on a pin of the other pin-conveyor wheel, to form strand array **16**. For each cycle of the traverse guide to and fro, each pin-conveyor wheel is advanced by the angular distance between successive pins. Strand array **16** is then carried by the rotating pin-conveyor wheels into contact with advancing sheet **12** as the sheet is fed from supply roll **13** onto and advanced by a sheet support surface, illustrated in the figures as rotating drum **50**. A short distance upstream ofthe location where strand array **16** contacts advancing sheet **12** an adhesive, preferably a melt adhesive, is applied to the strand array and/or the sheet by spraying, dripping, or other means. In Figures 1, 5 and 7, the adhesive is applied through adhesive spray applicator **20**. The strand array is carried by continued rotation of the pin-conveyor wheels into one or more nips located near each edge of the strand array. The nips are formed by restraining belts **52**, **52'**, preferably V-belts, and the circumferential surface of rotating drum **50**. Then, strand array **16** is removed from the pins and held by the restraining belts atop advancing sheet **12** on the drum surface. Optionally, a cover sheet **14** fed from supply roll **15** may be applied atop the just-formed strand array/sheet assembly. By setting the traverse speed and the pin spacing on each pin conveyor, and then adjusting the relative speeds of the pin conveyors and the sheet-advancing support, the number of transverse strands per unit length of composite sheet material produced can be varied as desired. As the assembled sheet substrate **12**, strand array **16** and optional cover sheet **14** are advanced, the adhesive becomes set and composite sheet material **18** is completed. Then, the composite sheet can be wound up or forwarded to further processing operations.

When the strand employed in the process or apparatus of the invention is an elastic strand (e.g., spandex), the resultant composite sheet material **18** can be cut in an optional subsequent step substantially parallel to the direction of the transverse strands to form elastic tapes or swatches. Then, the elastic tapes or swatches can be attached as elastic waistbands, side panels, closure tapes, frontal tapes, back panels and leg bands to disposable diapers and adult incontinence articles. High speed equipment of the kind disclosed by Merkatoris et al, U.S. Patent 5,296,080, is suitable for the attachment of the elastic components. Composite sheet material **18** formed by the process of the invention with strand that is not elastic is useful as strand-reinforced fabric, film, laminate, and the like . In such uses, strand **10** preferably is made of high tenacity fibers or filaments of polymers such as nylon, aramid, polyolefin, or polyester, or of glass or the like.

As used herein the term "strand" includes any monofilament, multifilament or staple yarn or thread. The strand can be of any decitex suitable for the application for which the resulting composite sheet material is intended. The strand can be made of synthetic or natural fibers. For elastic composites, the strand can be of natural or synthetic materials such as rubber, spandex or other elastomeric material. The term "spandex" has its usual generic meaning; namely, fiber made from a long chain synthetic polymer that comprises at least 85% by weight segmented polyurethane. Preferably, the spandex is employed with no lubricating finish on its surface so that better adhesion can be obtained between the spandex and the sheets.

Sheets suitable for use in the invention as advancing sheet substrate **12** include film, woven fabric , knit fabric or nonwoven fabric. The fabrics can be of natural or synthetic fibers such as cotton, wool, polyester, nylon, polypropylene, polyethylene, or the like. The films can be of polyethylene, polyester, polyfluorocarbons, polyimide, polypropylene, or the like.

Composite sheet materials **18** made by use of the process and apparatus of the invention typically have desirable resistances to impact, puncture and tear. Properties of the composite sheet can be further enhanced by processing the composite sheet material through the apparatus again one or more times. Uses for composite sheet material produced by the invention include tarpaulins, cargo curtains, bags, inflatable structures, hospital gowns, disposable coveralls, and the like. In some uses, elastomeric and reinforcing strands can be used advantageously together.

Various embodiments of the apparatus of the invention will now be described in further detail.

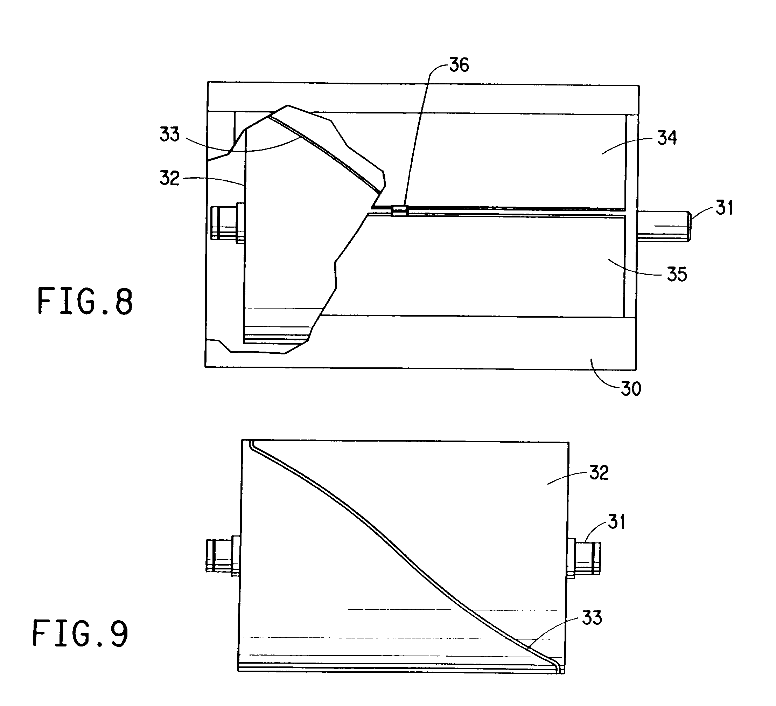

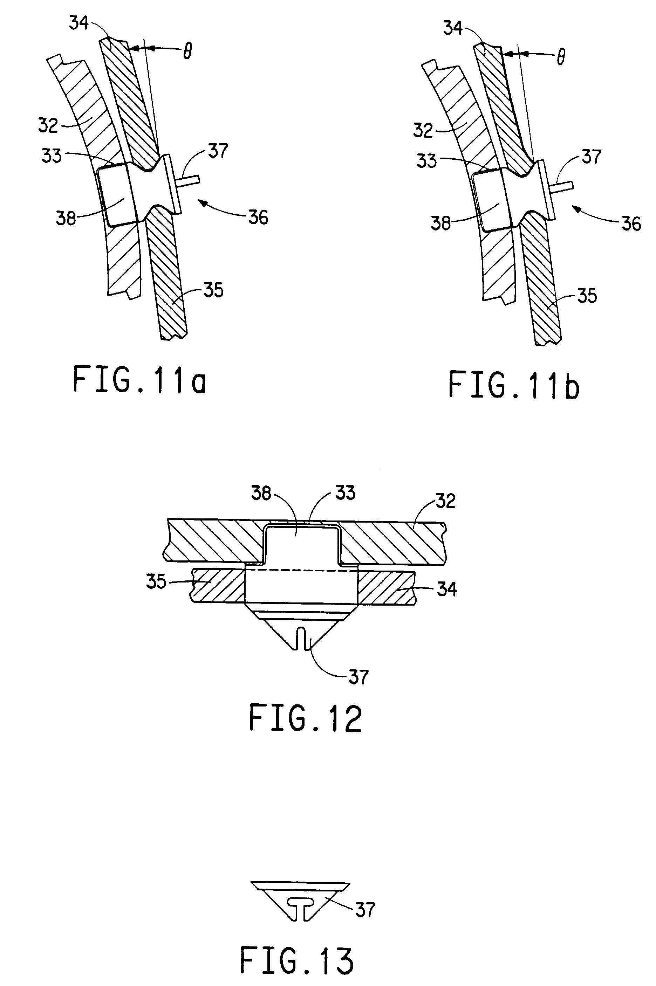

Cam/traverse-guide housing **30** encloses rotatable cylindrical cam **32**. Housing **30** also supports upper and lower guide rails **34**, **35**, between which strand traverse guide **36** is mounted. Housing **30** also prevents lubricating oil used on the cam from spraying into the work area. A 30-weight oil is suitable for lubricating the cam. Figure 8 is a front view of housing **30**, with a portion cut away to show cylindrical cam **32** within. Cylindrical cam **32** has groove **33** cut into its surface. The cylindrical cam is rotated by means not shown through cam shaft **31**. As shown by Figs. 11a, 11b and 12, strand traverse guide **36** comprises a tip **37** for holding the strand and a base **38** which acts as a cam-follower. The base or cam-follower portion **38** of strand traverse guide **36** is seated in cam groove **33** and slides through the groove path as cylindrical cam **32** is rotated. Rotation of grooved cylindrical cam **32** causes strand traverse guide **36** to slide to and fro between the straight edges of upper and lower guide rails **34** and **35**. Figure 9 is a front view detail of the cylindrical cam **32** with groove **33** in which the strand traverse guide cam follower **38** is to be seated. An access notch, not shown, can be provided in one of the guide rails for convenient installation of the strand guide in the cam groove and between the guide rails. A lead-in channel, not shown, connected to the groove in the circumferential surface of the cam, in a position that can be aligned with the access notch in the guide rail, permits convenient seating and removal of the strand guide.

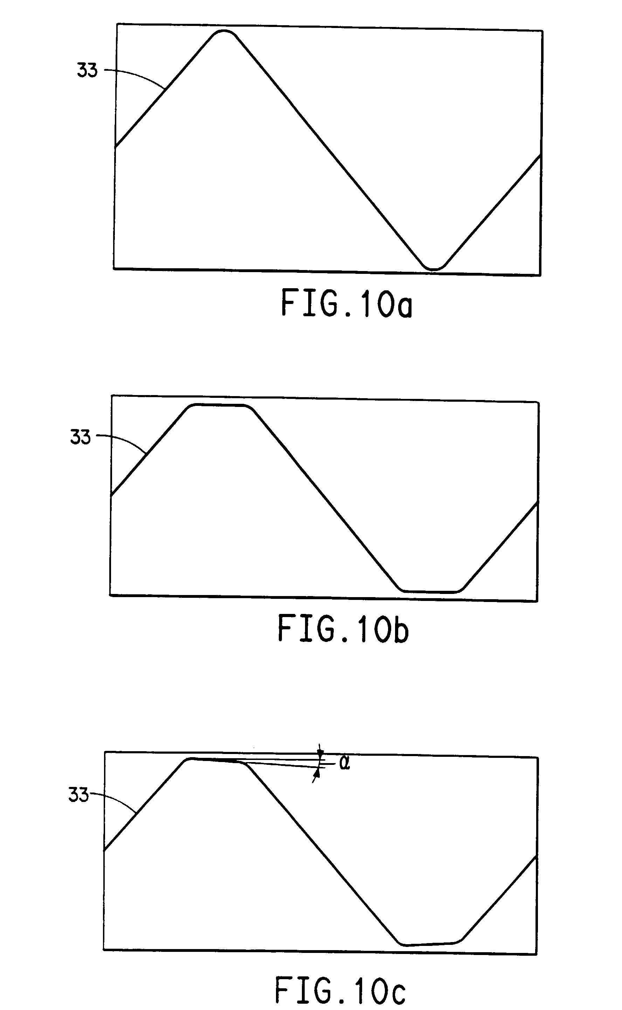

Figures 10a, 10b and 10c show three developed surfaces or "profiles" of cams for use in the apparatus of the invention. Each profile represents the complete surface of a cylindrical cam laid out in a plane. The curve represents groove **33** of cylindrical cam **32**. In operation, the cam follower of the strand traverse guide follows the groove path. The profile represents one rotation of the cam and results in one to and fro traverse of the strand traverse guide. Alternatively, suitable cams can have more than one rotation of the cam resulting in one to-and-fro traverse of the strand traverse guide. In following the groove of the cam profile shown in Figure 10a, the strand guide reverses direction immediately upon reaching the extreme end of its traverse. This cam is very similar to the cam disclosed in Akers et al. U.S. Patent 3,675,863. The flattened portions of the profile of Fig. 10b generate some "dwell time" at the extreme ends of the traverse by flattening of the profile. The dwell time increases the clearance of the strand guide around the pins of the pin-conveyor wheels and allows higher process speeds to be attained. Accordingly the profile of Fig. 10b is preferred over the profile of Fig. 10 a, which provides no dwell time at the point of traverse reversal. A further improvement in cam profile is shown in Figure 10c, in which a small angle, α, of less than 1 degree, is created between the flattened dwell portion of the profile and the edge of the cam. This maintains a small side pressure on the yarn guide so that it smoothly enters the turn at the end of the dwell section, thereby reducing wear on the guide and permitting higher speeds.

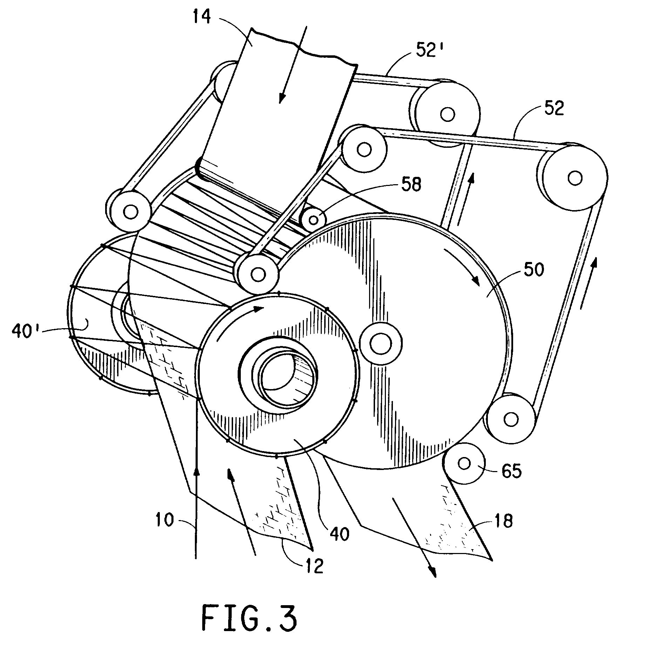

Figs. 11a, 11b and 12 show in greater detail cam follower **38** and tip **37** of strand traverse guide **36**. The cam follower is seated to slide within groove **33** of cylindrical cam **32** causing guide **36** to slide to and fro between guide rails **34** and **35**. In operation, as shown in Figs. 1-6, as cylindrical cam **32** is rotated, strand traverse guide **36** moves strand **10** to and fro to loop the strand about pins **42**, **42'** of pin-carrying conveyors **40**, **40**'. Pins **42**, **42**' are driven in a semicircular path that sweeps through the path of the strand traverse guide (after the guide has passed) to engage the strand **10**. Figs. 6 and 11a depict guide rails **34**, **35** as flat. Fig. 11b depicts an upper guide rail **34**, preferred for bottom-fed strand, having a shallow depression or groove in its surface at the region of closest approach of the pins **42**, **42'** to rail **34**. This permits the pins to sweep more deeply through the strand traverse path without hitting the guide rail. Also, as shown in Figs. 11a and 11b, for bottom fed strand, upper rail **34** is recessed by an angle θ, usually of less than 10 degrees, to further assure a close, unimpeded approach of the pins to the strand traverse path. The close approach of the pins to the face of the guide rails assures that as the pins contact the strand, the strand is secure in the notch of the traverse guide tip. Details of the notched tip of the strand guide are depicted in Figs. 12 and 13. Such notched guide tips are disclosed by Altice et al, U.S. Patent 3,086,722. If the strand is fed from above the apparatus, the central axis of the semicircular path of the pins preferably is positioned slightly below the path of strand traverse guide, and the hollows or grooved areas are on the lower guide rail to accommodate the path of the pins.

In a system tested by the inventors, tip **37** of the guide **36** extended about 2.5 mm (0.098 inch) above the base of the strand guide and had a notch about 1.5 mm (0.060 inch) deep. Of course, guides of other dimensions can be used satisfactorily. Figure 13 illustrates a key-hole shaped notch for the tip of the strand traverse guide . The key-hole notched tip is preferred for better retention of the strand in the guide. To minimize wear and friction, tip **37** preferably is constructed of a ceramic material having a surface roughness no greater than 8x10⁻⁴mm (32 micro inches) RMS. When a plastic guide is employed, the ceramic tip can be integrally molded with the plastic to form the completed guide. For smoothest operation, the weight of the traverse guide weight is minimized. A typical plastic traverse guide with ceramic tip can weigh as little 5 to 10 grams.

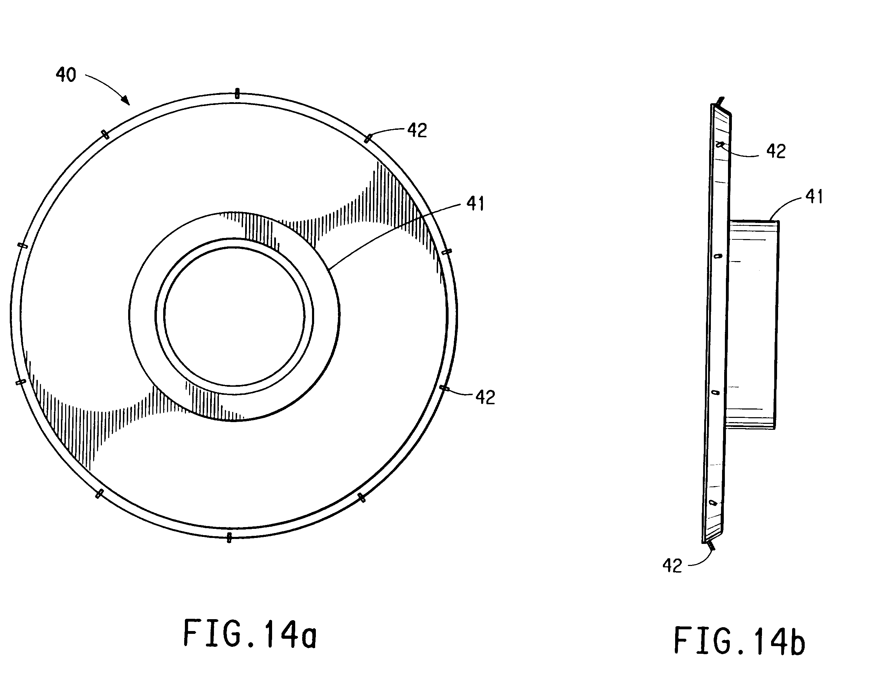

As shown in Figs. 1-5, strand 10, carried by reciprocating strand traverse guide **36**, is looped around pins **42**, **42'** of a pair of pin-carrying conveyors **40**, **40'** to form a strand array. As illustrated in Figs 1-5, the pin-carrying conveyors are wheels, each having a plurality of pins projecting from its surface. A pin-carrying conveyor wheel is located near each end of the guide traverse path. The pin-conveyor wheels are spaced apart a distance that is somewhat less than the full stroke of the traverse guide. As referred to herein, the stroke of the traverse guide is equal to one-half the length of the full to-and-fro traverse of the strand traverse guide. Figs. 14a and 14b respectively depict a typical pin-conveyor wheel **40** with a plurality of pins **42** projecting outwardly from the wheel. The wheel has an central annular cylindrical hub **41** for mounting a drive shaft (not shown). The pins can be press-fit, brazed, welded, or bonded into spaced holes in the wheels. Typically, the are rotated at a rate in the range of 100 to 600 revolutions per minute; at least 200 rpm is preferred.

In another embodiment of the pin wheels, to ease strand removal from the pins, the pins can be made retractable, as for example by a cam and spring mechanism. As illustrated in Figures 1 and 5 for bottom-fed strand, the axes of the pin-conveyor wheels are preferably positioned slightly above (for example, by about 2.5 mm) the elevation of the strand traverse guide path to allow deeper penetration of the pins across the path of the strand guide. As illustrated in Fig. 4 for top fed strand, the axes of the pin-conveyor wheels are positioned slightly below the elevation of the strand traverse guide. Pin-conveyor wheels **40**, **40'** are driven by conventional means not shown in synchronization with the traverse guide. The pin-conveyor wheels are spaced from each other by a distance that is shorter than the traverse guide stroke but sufficiently long to permit restraining belts 52, 52' to capture the ends of the strands. The angular location of pins on the circumference of one wheel is off-set from the angular position of the pins on the circumference of the other wheel by one-half of the pin spacing. For high speed looping of strand around the pin of the pin conveyors, a minimum clearance 1mm (0.040inches) between the strand traverse guide and the pins of the pin-conveyor wheels has been found to be satisfactory.

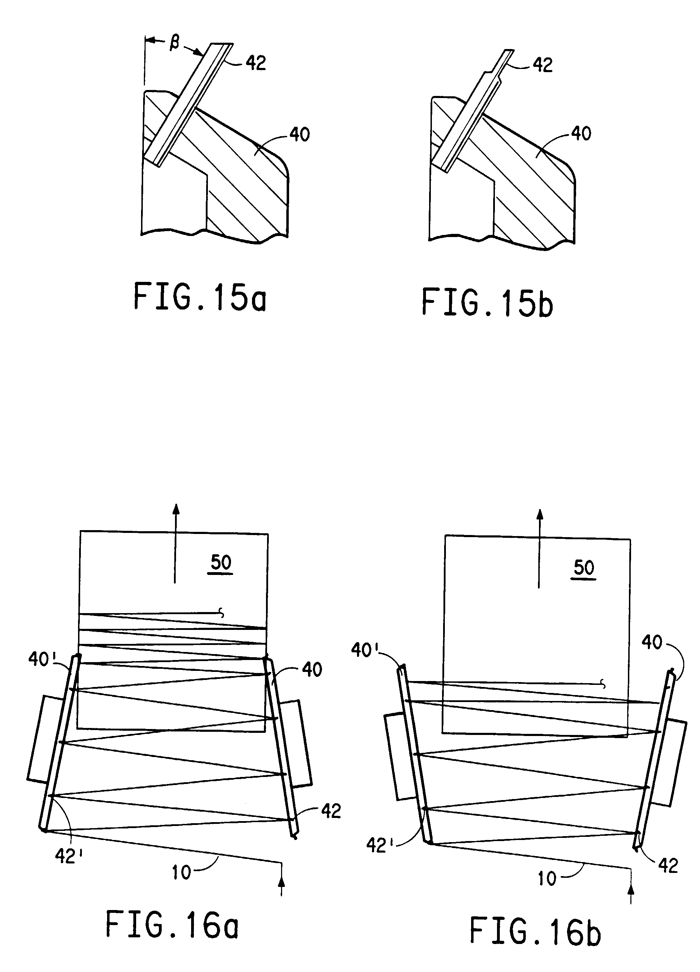

The detail drawings of Figs. 15a and 15b, show a pin **42** canted at an angle β from the plane of wheel **40** and projecting from the surface of the pin-conveyor wheel. A typical pin can project 6.4mm (0.25 inch) from the surface. The pin is directed slightly away from the centre of the strand traverse path to improve the ability of the pin to hold the strand securely in place on the wheel. Angle β can be as large as 45 degrees, but usually is smaller. As illustrated in Fig. 15b, the pin has a shoulder about 2/3 of the distance from the point at which the pin is secured to the wheel to the exposed end of the pin. A shoulder located between 0.4 and 0.8 of said distance usually ensures satisfactory looping of the strand.

In Figs. 16a and 16b respectively, the pin-conveyor wheels **40**, **40'** are schematically shown to be canted toward or away from each other. Such arrangements are particularly useful with elastomeric strand. As shown in Figure 16a, strand **10** is formed into a looped strand array on the pin-conveyor wheels, and the canted wheels allow the strand to retract before the strand is laid down on the sheet substrate carried by sheet support drum **50**. In Figure 16b, strand **10** is formed into a looped array on the pins of the pin-conveyor wheels and then stretched by the canted wheels before being laid down on the moving sheet. With this latter configuration, a shorter traverse stroke and therefore higher speeds can be used. While on the pins, a spandex strand typically can have an elongation that is in the range of 10 to 400% beyond its relaxed length.

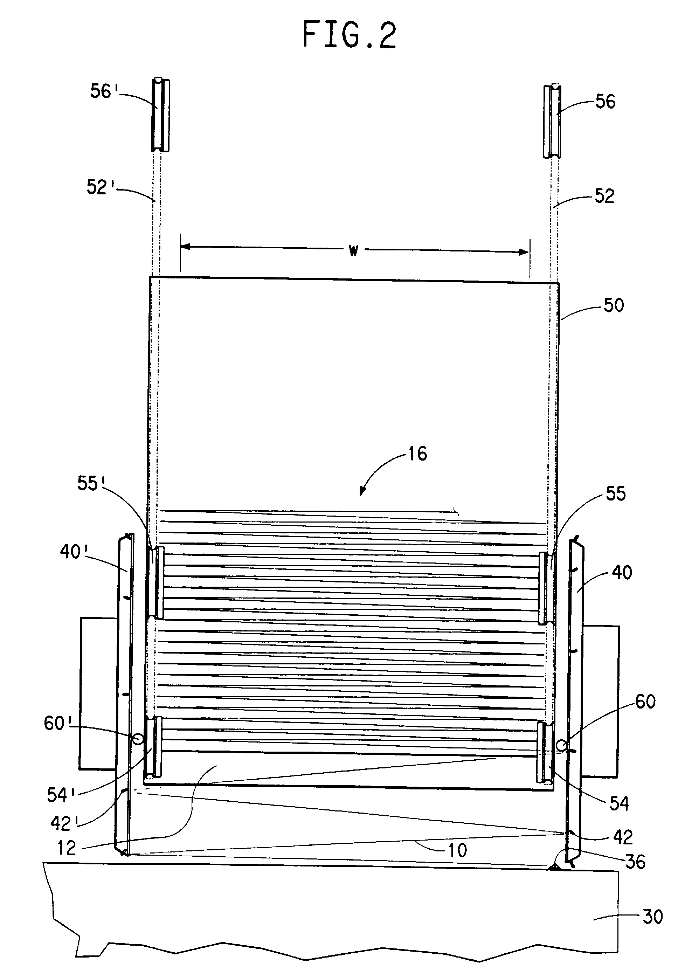

As illustrated in Figs. 1-5, sheet **12**, which is to become the substrate of the composite sheet material **18** to be produced by the invention, is advanced on the surface of sheet support drum **50**. Drum **50** is positioned so that at least a part of its circumferential surface is between pin-conveyor wheels **40**, **40'**. Other sheet support surfaces contemplated for use in supporting the sheet in its passage between the pin-conveyor wheels, include for example, an endless flat belt or screen. An endless belt is advantageous when additional time is required for maintaining the strand array and sheet assembly restrained while the adhesive is set. To keep laid-down strand array **16** in position atop advancing sheet substrate **12**, a pair of restraining belts **52**, **52'** are employed. Restraining belt **52** is driven by contact with the moving sheet support drum around a set of four idler rolls **54**, **55**, **56** and **57**; belt **52'** is driven in the same way around corresponding idler rolls **54'**, **55'**, **56'** and **57'**. Idler rolls **54**, **54'** are positioned so that strand array **16** is initially captured at the nip between the belts going around rolls **54**, **54'** and rotating sheet support drum **50** before the strand looped around pins **42**, **42'** of pin-conveyor wheels **40**, **40'** is removed from the pins. Drum **50** can have circumferential grooves aligned with the restraining belts to aid the belts in holding the advancing strand array and sheet in position on the drum. Idler rolls **54**, **54'** preferably are of small diameter so that the distance between the point at which the strand is contacted by the restraining belts and the point at which the strand is fully captured in the nip between the belts and the drum is as small as practicable. Preferably located immediately upstream of the nips between the restraining belts and the drum, is adhesive applicator **20**, which applies adhesive to the strand and/or advancing sheet. A wide variety of adhesives are suited for use in the apparatus, though hot melt spray adhesives are preferred. The width of sheet affected by the adhesive applied to the strand and/or sheet is designated "W" in Fig. 2 and is a little shorter than the distance between the restraining belts.

Restraining belts **52**, **52'** are preferably V-belts and more preferably grooved V-belts. Such belts hold the strand and sheet on the sheet support drum more securely than do belts of round or flat cross-section. The belts are made of flexible materials to permit proper passage of the belt around the smallest diameter idler rolls (i.e., **54**, **54'**). Restraining belts **52**, **52'** operate at the same linear speed as the circumferential speed of sheet support drum **50**. Immediately downstream of the just-described belt/drum nips, cutters **60**, **60'** are positioned on each side of drum **50** (as best illustrated in Fig. 2 and 7). Various types of cutters can be used, such sharp edges, hot wire cutters (i.e., electrically heated resistance elements), or any other means suitable for the particular kind of strand being cut. Instead of employing cutters, stripping fingers may be positioned so as to lift the loops of strand off the pins after the strand is engaged by the restraining belts. Using stripping fingers leaves loops at the selvage of the final composite sheet product. Optionally, a press roll **58** can be used to press a cover sheet **14**, supplied from roll **15**, atop restrained assembly of strand array **16** and sheet substrate **12**, while the assembly is still on the support drum and before the applied adhesive has set. A takeoff roll **65** can be used to guide the completed composite sheet material away from the drum and to a suitable collection means or to further processing steps. The assembly of strand, sheets and adhesive are restrained on the drum for a sufficient time (or distance on the drum) to permit the adhesive applied to the strand and sheet to set.

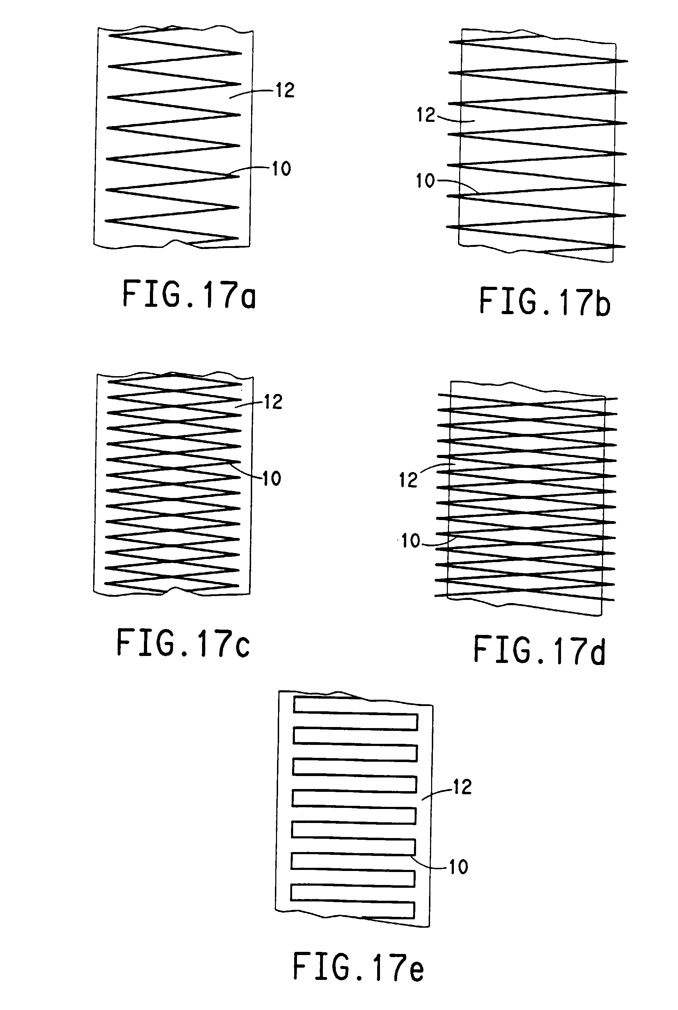

Figs. 17a-e show various patterns of strand array **16** on substrate sheet **12** that can be made with the present invention. The array of Fig. 17a is prepared with the apparatus herein before described with regard to Figs. 1 and 2. Fig. 17b is a similar pattern but the ends of the strand **10** extend beyond the edges of the sheet **12**. This pattern also can be made if the distance between the pin-conveyor wheels is shorter than the axial length of the sheet support drum and the width of the substrate sheet. Figs. 17c and 17d show patterns comparable to 17a and 17b, but with two strands **10** having been laid down on a sheet **12**. To make these latter patterns, one pair of a pin-conveyor wheels is used in conjunction with two cam-driven strand traverse guides to loop the strands onto the pins of the pin-conveyor wheels. The pattern of Figure 17e is made when one employs non-round (i.e., oblong) pins which rotate after having had strand looped around them. The narrow profile of the pins is presented during the strand looping. Rotation of the pins after the strand looping "expands" the machine-direction length of the strand and results in a strand array in which the strand segments traversing the final composite sheet material are more parallel to each than when stationary pins of round cross-section are used.

### EXAMPLE

This example describes a test that demonstrates the suitability of the process and apparatus of the invention for making elastic composite material at high speed and installing the material as elastic component in a garment, in particular an elastic waistband in disposable diapers. The apparatus for making the elastic composite sheet material used for the waistbands is substantially as illustrated in Fig. 1. The process also can be carried out using other embodiments of the invention.

The starting materials are as follows. A 620-dtex LYCRA® XA™ spandex (sold by E. I. du Pont de Nemours & Co., Wilmington, DE) is used as elastic strand **10**. A 23.3-g/m² Type 6700 thermally bonded polypropylene nonwoven fabric (sold by Fiberweb Group Inc., Simpsonville, NC) is used as substrate sheet **12**. a 0.025mm (0.001 inch) thick polyethylene film (sold by Consolidated Thermoplastics Co., Dallas, TX) is used as cover sheet **14**. Findley 2276 hot melt adhesive (sold by Findley Adhesives, Inc., Wauwatosa, WI) is sprayed onto the strand and substrate sheet to obtain a total adhesive loading of 1.92 mg/cm² (12 mg/in²) in the final composite sheet material.

The strand is fed under tension to reciprocating strand guide which is driven by a cylindrical cam rotating at 2,000 rpm (rotations per minute). The strand is supplied at an extension of about 300% beyond its relaxed length. The strand traverse guide has a 23.5cm (0.5 inch) stroke and makes 2,000 to-and-fro cycles per minute. A pair of parallel pin-conveyor wheels, 22.2cm (8.75 inches) apart (i.e., the minimum distance between the imaginary circles formed by the centers of the bases of the pins on each wheel) and each of 16.2cm (6.37 inch) diameter are rotated at 200 rpm. Each pin-conveyor wheel has ten pins equally spaced apart by a circumferential distance of 5cm (2.0 inches). The pins are canted outward by 20 degrees from the wheel circumferential surface. The cam profile is as given in Figure 10b. The positions of the pin wheels and strand traverse guide are set to provide a clearance of at least 1 mm (0.04 inch) between the pins and the strand traverse guide near the end of each traverse stroke. The axes of the pin-conveyor wheels are located 3.8mm (0.15 inches) above the elevation of the axis of the path of the traverse guide. Fig. 15b illustrates the configuration that is used for the pins and the pin-conveyor wheels. Each pin is of circular cross-section and has a shoulder of the design illustrated in Fig. 15b. The pins project above the wheel surface. The pin shoulder is located 1.8mm (0.07 inch) from the exposed tip of the pin. The large diameter portion of the pin is 1.5mm (0.060 inch) and the small diameter portion (i.e., the portion closest to the exposed tip of the pin) is 1 mm (0.038 inch). The sheet support drum has a 23.5cm (9.5 inch) diameter and is rotated at 25 rpm. The resultant composite sheet material is removed from the drum at a linear velocity of about 19 metres per minute.

The composite sheet material is then fed to a commercial Nuova Red diaper-making machine having a belt-type waistband applicator. The elastic composite sheet material is fed, cut, vacuum-transferred and glued into the disposable diapers to form satisfactory waistbands in the diapers at a rate of about 300 diapers per minute.

The preceding example illustrated the use of elastic composite sheet material of the invention in waistbands of disposable diapers. The elastic composite sheet is also suited for installation in adult incontinence articles as waistbands, and in diapers and adult incontinence articles as stretch side panels, closure tapes, frontal tapes, back panels, leg bands, and the like. The elastic composite sheet material is also suited for installation in portions of other types of garments, such as in wrist bands of sweaters, waist bands of trousers, elastic portions of athletic sportswear, as well as many other articles of clothing.

## Claims

1. A process for making a composite sheet material, wherein the process comprises
advancing in a longitudinal direction a sheet substrate (2), a first conveyor and second conveyor (40,40'), the sheet substrate having two lateral edges and an upper and a lower surface and each conveyor (40,40') having a plurality of spaced apart pins (42,42') extending therefrom in a direction generally perpendicular to the movement of the conveyors, the conveyors being spaced apart and operating in synchronization,
supplying a strand (10) to a device that reciprocates the strand to and fro across a traverse path, the traverse path being generally transverse to the longitudinal direction of the advancing sheet substrate (12),
advancing the conveyors (40,40') to move the pins (42,42') through a path that intersects the traverse path of the strand (10), thereby causing the strand to repetitively loop alternately, at each end of the traverse path, around a pin on the first conveyor and then around a pin on the second conveyor to form a strand array (16) having edges carried by the pins,
applying an adhesive to the strand array (16) or to the advancing sheet substrate (12) or to both the strand array (16) and the advancing sheet substrate (12),
transferring the strand array (16) from the pins (42,42') to the upper surface of the advancing sheet substrate (12) to form a strand-sheet assembly, and forwarding the resultant composite sheet material (18) to a windup or to further processing steps,
characterized in that the process further comprises:
feeding the strand (10) to a strand traverse guide (36) driven by a rotating cylindrical cam (32),
moving the pins through an arcuate path that intersects the traverse path of the strand,
advancing the sheet substrate on a moving sheet support, and
restraining each edge of the strand array (16) in position on the sheet substrate from a location after the point where the strand array is transferred from the pins to the advancing sheet substrate to a location beyond the point where the applied adhesive has become set.

2. A process according with claim 1, further comprising adhering a cover sheet (14) atop the strand-sheet assembly.

3. A process in accordance with claim 1 or 2, wherein the strand is reciprocated at a rate in the range of 250 to 3,000 to-and-fro cycles per minute with a traverse stroke of at least 5-centimetre length, the pin-carrying conveyors are moved at an equal speed and are rotated at a rate of 100 to 600 revolutions per minute through a semicircular path that intersects the traverse path of the strand.

4. A process in accordance with claim 3 wherein the strand is an elastic strand that is elongated in the range of 10 to 400% beyond its original relaxed length while being supplied to the strand traverse guide and/or while being restrained in the strand array.

5. A process in accordance with claim 1, 2, 3 or 4 wherein the sheet substrate (12) and the optional cover sheet (14) are each a nonwoven fabric.

6. A process in accordance with claim 1, 2, 3 or 4 wherein the sheet substrate (12) is a nonwoven fabric and the optional cover sheet (14) is a film.

7. A process in accordance with any preceding claim wherein the strand (10) is a spandex and the resultant composite sheet material is cut into lengths and attached as elastic components of a garment.

8. A process in accordance with claim 7 wherein the composite sheet material is cut into tapes or swatches and supplied to an elastic swatch applicator of a diaper-making machine to adhere the cut tapes or swatches to the diapers to form elastic bands in the diapers.

9. An apparatus for making a composite sheet material in which a strand (10) supplied through a reciprocating guide is looped around pins (42,42') of a pair of spaced-apart pin conveyors (40,40'), the pin conveyors transfer the strand transversely to a sheet substrate (12) advancing in a longitudinal direction and the strand is adhered to the sheet substrate,
characterized in that:
the reciprocating guide is a lightweight strand guide (36) connected to a cylindrical cam (32), the strand guide having a slotted tip (37) for receiving the strand and a follower portion (38) for fitting a groove (33) in the cylindrical cam (32), so that when the cam is rotated the strand guide is driven to and fro along a traverse path having a first end and a second end;
the pin conveyors each have a plurality of evenly spaced-apart pins (42,42') projecting from the surface of the conveyor, the first conveyor being positioned near the first end of the traverse path and the second conveyor being positioned near the second end of the traverse path, and the space between the conveyors being less than the distance between the first and second ends of the traverse path;
means for moving the pins of the conveyor through an arcuate path that intersects the traverse path, so that during operation strand carried by the reciprocating guide, near each end of each traverse, is looped around a pin of the pin conveyor;
a surface (50) for supporting and moving the sheet substrate in the longitudinal direction into a position for transferring the strand from the pins to the sheet substrate, the transfer position being located between the pin conveyors and at a nip formed by a restraining means (52,52') and the support surface;
means for synchronizing the relative speeds of the reciprocating traverse guide and the pin conveyors to assure the looping of strand on a pin of the conveyor at each end of the strand path; and
means for controlling the movement of the restraining means and the support surface at equal speeds.

10. An improved apparatus of claim 9 wherein each pin conveyor is a rotatable wheel pin conveyor; the support surface is a rotatable cylindrical drum; and the restraining means is a pair of V-belts.

11. An improved apparatus of claim 10 wherein the slot of the strand guide is a key-hole slot and each pin of each pin-conveyor wheel has a shoulder located between 0.4 and 0.8 of the distance from where the pin is secured to the wheel to the top of the pin and separating a larger diameter portion of the pin from a smaller diameter portion of the pin, the smaller diameter portion being the top portion of the pin.

12. An improved apparatus of claim 10 or 11 wherein the pin-conveyor wheels are canted toward each other or away from each other.

## Patentansprüche

1. Verfahren zum Herstellen eines Verbundschichtmaterials mit den folgenden Schritten:
Vorwärtsbewegen eines Lagen- bzw. Schichtsubstrats (2), einer ersten Fördereinrichtung und einer zweiten Fördereinrichtung (40, 40) in einer Längsrichtung, wobei das Schichtsubstrat zwei Seitenkanten sowie eine obere und untere Fläche besitzt und jede Fördereinrichtung (40, 40) mehrere beabstandete Stifte (42, 42), die sich in einer im wesentlichen senkrechten Richtung zur Bewegung der Fördereinrichtungen von ihr erstrecken, besitzt, und wobei die Fördereinrichtungen beabstandet sind und synchron laufen,
Bereitstellen einer Litze bzw. eines Fadens (10) für eine Vorrichtung, die den Faden bzw. die Litze über einen Querpfad hin- und herbewegt, wobei sich der Querpfad im wesentlichen quer zur Längsrichtung des vorwärtsbewegenden Schichtsubstrats (12) erstreckt,
Vorwärtsbewegen der Fördereinrichtungen (40, 40), um die Stifte (42, 42) durch einen Pfad, der den Querpfad des Fadens (10) schneidet, zu bewegen, wobei der Faden veranlaßt wird, sich wiederholt und abwechselnd an jedem Ende des Querpfads um einen Stift auf der ersten Fördervorrichtung und dann um einen Stift auf der zweiten Fördervorrichtung zu schlingen, um eine Fadenanordnung (16) mit von den Stiften getragenen Kanten zu bilden,
Aufbringen eines Klebstoffs auf die Fadenanordnung (16) oder auf das sich vorwärtsbewegende Schichtsubstrat (12) oder sowohl auf die Fadenanordnung (16) als auch auf das sich vorwärtsbewegende Schichtssubstrat (12),
Übertragen der Fadenanordnung (16) von den Stiften (42, 42) auf die obere Fläche des sich vorwärtsbewegenden Schichtsubstrats (12), um einen Fadenschichtaufbau zu bilden, und Weiterleiten des resultierenden, zusammengesetzten Schichtmaterials (18) zu einer Aufrolleinrichtung oder zu weiteren Verarbeitungsschritten,
gekennzeichnet durch,
Einführen des Fadens (10) in eine Fadenquerführung (36), die durch einen sich drehenden, zylindrischen Betätigungsvorsprung (32) angetrieben wird,
Bewegen der Stifte durch einen bogenförmigen Pfad, der den Querpfad des Fadens schneidet,
Vorwärtsbewegen des Schichtsubstrats auf einem sich bewegenden Schichtträger und
Fest- bzw. Zurückhalten jeder Kante der Fadenanordnung (16) in Position auf dem Schichtsubstrat von einem Ort nach der Stelle, wo die Fadenanordnung von den Stiften auf das sich vorwärtsbewegende Schichtsubstrat übertragen wird, auf einen Ort jenseits der Stelle, wo der aufgebrachte Klebstoff festgeworden ist.

2. Verfahren nach Anspruch 1, weiterhin mit Anheften einer Deckschicht (14) auf dem Fadenschichtaufbau.

3. Verfahren nach Anspruch 1 oder 2, wobei der Faden mit einer Geschwindigkeit im Bereich von 250 bis 3000 Hin- und -Her-Zyklen pro Minute mit einem Querhub von mindestens 5 cm Länge hin- und herbewegt wird, wobei die stifttragenden Fördereinrichtungen mit gleicher Geschwindigkeit bewegt und mit einer Geschwindigkeit von 100 bis 600 Umdrehungen pro Minute durch einen halbkreisförmigen Pfad, der den Querpfad des Fadens schneidet, gedreht werden.

4. Verfahren nach Anspruch 3, wobei der Faden ein elastischer Faden ist, der im Bereich von 10 bis 400% über seine ursprüngliche, entspannte Länge gestreckt wird, während er in die Fadenquerführung geschickt und/oder während er in der Fadenanordnung festgehalten wird.

5. Verfahren nach Anspruch 1, 2, 3 oder 4, wobei das Schichtsubstrat (12) und die optionale Deckschicht (14) nicht gewobene Stoffe bzw. Waren sind.

6. Verfahren nach Anspruch 1, 2, 3 oder 4, wobei das Schichtsubstrat (12) ein nicht gewobener Stoff und die optionale Deckschicht (14) ein Film ist.

7. Verfahren nach einem der vorhergehenden Ansprüche, wobei der Faden (10) ein Spandex ist und das resultierende, zusammengesetzte Schichtmaterial in Abschnitte geschnitten und als elastische Komponenten auf ein Kleidungsstück aufgebracht wird.

8. Verfahren nach Anspruch 7, wobei das zusammengesetzte Schichtmaterial in Streifen oder Musterabschnitte geschnitten und an eine Auftragseinrichtung für elastische Muster einer Windelherstellungsmaschine geschickt wird, um die geschnittenen Streifen oder Muster an die Windeln anzuheften, damit in den Windeln elastische Bänder ausgebildet werden.

9. Vorrichtung zum Herstellen eines Verbundschichtmaterials, in der ein Faden bzw. eine Litze (10), die durch eine sich hin- und herbewegende Führung bereitgestellt wird, um Stifte (42, 42) eines Paars von beabstandeten Stiftfördereinrichtungen (40, 40) geschlungen wird, wobei die Stiftfördereinrichtungen den Faden quer zu einem sich in einer Längsrichtung vorwärtsbewegenden Schichtsubstrat (12) übertragen und der Faden an das Schichtsubstrat angeheftet ist,
dadurch gekennzeichnet, daß
die sich hin- und herbewegende Führung eine leichtgewichtige Fadenführung (36) ist, die mit einem zylindrischen Betätigungsvorsprung (32) verbunden ist, wobei die Fadenführung eine geschlitzte Spitze (37) zum Aufnehmen des Fadens und einen Folgerabschnitt (38) zum Einpassen in eine Vertiefung (33) in dem zylindrischen Betätigungsvorsprung (32) besitzt, so daß, wenn der Betätigungsvorsprung gedreht wird, die Fadenführung hin und her entlang eines Querpfads mit einem ersten und einem zweiten Ende getrieben ist,
die Stiftfördereinrichtungen jeweils mehrere gleich beabstandete Stifte (42, 42), die von der Oberfläche der Fördereinrichtung vorspringen, besitzen, wobei die erste Fördereinrichtung nahe dem ersten Ende des Querpfads und die zweite Fördereinrichtung nahe dem zweiten Ende des Querpfads positioniert ist und der Abstand zwischen den Fördereinrichtungen geringer als die Entfernung zwischen dem ersten und zweiten Ende des Querpfads ist,
eine Einrichtung zum Bewegen der Stifte der Fördereinrichtung durch einen bogenförmigen Pfad, der den Querpfad schneidet, so daß während des Betriebs der durch die sich hin- und herbewegende Führung geführte Faden in der Nähe jeden Endes jeder Durchquerung um einen Stift der Stiftfördereinrichtung geschlungen ist,
eine Oberfläche (50) zum Tragen und Bewegen des Schichtsubstrats in der Längsrichtung in eine Position zum Übertragen des Fadens von den Stiften auf das Schichtsubstrat, wobei sich die Übertragungsposition zwischen den Stiftfördereinrichtungen und bei einer dünnen Stelle bzw. Nip oder einem Einlaufspalt, die/der durch eine Rückhalteeinrichtung (52, 52) und die Trägeroberfläche gebildet ist, befindet,
eine Einrichtung zum Synchronisieren der relativen Geschwindigkeiten der sich hin- und herbewegenden Querführung und der Stiftfördereinrichtungen, um das Umschlingen des Fadens um einen Stift der Fördereinrichtung an jedem Ende des Fadenpfads sicherzustellen, und
eine Einrichtung zum Steuem bzw. Regeln der Bewegung der Rückhalteeinrichtung und der Trägeroberfläche bei bzw. auf gleichen Geschwindigkeiten vorgesehen sind.

10. Verbesserte Vorrichtung nach Anspruch 9, wobei jede Stiftfördereinrichtung eine drehbare Radstiftfördereinrichtung, die Trägeroberfläche eine drehbare, zylindrische Trommel und die Rückhalteeinrichtung ein Paar von V-Gurten ist.

11. Verbesserte Vorrichtung nach Anspruch 10, wobei der Schlitz der Fadenführung ein Schlüssellochschlitz ist und jeder Stift von jedem Stiftförderungseinrichtungsrad eine Schulter besitzt, die sich zwischen 0,4 und 0,8 von der Entfernung, von wo der Stift an das Rad befestigt ist zur Spitze des Stifts befindet und einen Abschnitt des Stifts mit größerem Durchmesser von einem Abschnitt des Stifts mit kleinerem Durchmesser trennt, wobei der Abschnitt mit kleinerem Durchmesser der Spitzenabschnitt des Stifts ist.

12. Verbesserte Vorrichtung nach Anspruch 10 oder 11, wobei die Stiftfördereinrichtungsräder hin zueinander oder weg voneinander abgeschrägt sind.

## Revendications

1. Procédé de fabrication d'un matériau en feuille composite, lequel procédé comprend les étapes consistant à :
faire progresser dans une direction longitudinale un substrat en feuille (2), un premier moyen d'acheminement et un deuxième moyen d'acheminement (40, 40'), le substrat en feuille comportant deux bords latéraux et des surfaces supérieure et inférieure, et chaque moyen d'acheminement (40, 40') comportant une pluralité de picots espacés les uns des autres (42, 42') s'étendant dans une direction généralement perpendiculaire au mouvement des transporteurs, les transporteurs étant espacés l'un de l'autre et fonctionnant en synchronisme;
fournir un brin (10) à un dispositif qui fait aller et venir le brin suivant un trajet de va-et-vient, le trajet de va-et-vient étant généralement transversal à la direction longitudinale du substrat en feuille en progression (12);
faire progresser les transporteurs (40, 40') pour déplacer les picots (42, 42') à travers un trajet qui croise le trajet de va-et-vient du brin (10), amenant de ce fait le brin à se boucler de manière répétitive et alternative, à chaque extrémité du trajet de va-et-vient, autour d'un picot sur le premier transporteur, puis autour d'un picot sur le deuxième transporteur pour former un arrangement de brin (16) présentant des bords portés par les broches;
appliquer un adhésif sur l'arrangement de brin (16) ou sur le substrat en feuille en progression (12) ou à la fois sur l'arrangement de brin (16) et le substrat en feuille en progression (12);
transférer l'arrangement de brin (16) des picots (42, 42') vers la surface supérieure du substrat en feuille en progression (12) pour former un ensemble brin-feuille, et envoyer le matériau en feuille composite obtenu (18) vers un dispositif d'envidage ou d'autres étapes de traitement, caractérisé en ce que le procédé comprend en outre les étapes consistant à :
amener le brin (10) à un guide-brin à mouvement de va-et-vient (36) entraîné par une came cylindrique rotative (32);
déplacer les picots suivant un trajet courbe qui croise le trajet de va-et-vient du brin;
faire progresser le substrat en feuille sur un support en feuille mobile, et
retenir chaque bord de l'arrangement de brin (16) en place sur le substrat en feuille, d'un emplacement après le point où l'arrangement de brin est transféré des picots vers le substrat en feuille en progression jusqu'à un endroit situé au-delà du point où l'adhésif appliqué a fait prise.

2. Procédé suivant la revendication 1, comprenant en outre l'étape consistant à coller une feuille de recouvrement (14) sur l'ensemble brin-feuille.

3. Procédé suivant la revendication 1 ou 2, dans lequel le brin est animé d'un mouvement de va-et-vient à une vitesse comprise entre 250 et 3000 cycles aller/retour par minute avec une course transversale d'au moins 5 cm de longueur, les transporteurs portant les picots sont déplacés à des vitesses égales et tournent à raison de 100 à 600 tours par minute suivant un trajet semi-circulaire qui croise le trajet de va-et-vient du brin.

4. Procédé suivant la revendication 3, dans lequel le brin est un brin élastique qui est étiré à raison de 10 à 400% au-delà de sa longueur au repos d'origine tout en étant fourni au guide-brin à mouvement de va-et-vient et/ou tout en étant retenu dans l'arrangement de brin.

5. Procédé suivant la revendication 1, 2, 3 ou 4, dans lequel le substrat en feuille (12) et la feuille de recouvrement facultative (14) sont chacun un tissu non tissé.

6. Procédé suivant la revendication 1, 2, 3 ou 4, dans lequel le substrat en feuille (12) est un tissu non tissé et la feuille de recouvrement facultative (14) est un film.

7. Procédé suivant l'une quelconque des revendications précédentes, dans lequel le brin (10) est de type Spandex et le matériau en feuille composite obtenu est coupé en longueurs et fixé à titre de composants élastiques d'un vêtement.

8. Procédé suivant la revendication 7, dans lequel le matériau en feuille composite est coupé en bandes ou en pièces et est fourni à un applicateur de pièce élastique d'une machine à fabriquer des couches pour coller les bandes coupées ou les pièces aux couches afin de former des bandes élastiques dans les couches.

9. Appareil de fabrication d'un matériau en feuille composite, dans lequel un brin (10) fourni à travers un guide à mouvement de va-et-vient est bouclé autour de picots (42, 42') d'une paire de transporteurs à picots espacés (40, 40'), les transporteurs à picots transfèrent le brin de manière transversale vers un substrat en feuille (12) progressant dans une direction longitudinale et le cordon est collé au substrat en feuille, caractérisé en ce que :
le guide à mouvement de va-et-vient est un guide-brin léger (36) relié à une came cylindrique (32), le guide-brin comportant un bout fendu (37) destiné à engager une gorge (33) dans la came cylindrique (32), de sorte que, lorsque la came tourne, le guide-brin est entraîné dans un sens puis dans l'autre le long d'un trajet de va-et-vient comportant une première extrémité et une deuxième extrémité;
les transporteurs à picots comportent chacun une pluralité de picots espacés les uns des autres de manière uniforme (42, 42') faisant saillie de la surface du transporteur, le premier transporteur étant positionné près de la première extrémité du trajet de va-et-vient et le deuxième transporteur près de la deuxième extrémité du trajet de va-et-vient, et l'espace entre les transporteurs étant inférieur à la distance entre les première et deuxième extrémités du crajet de va-et-vient;
des moyens permettent de déplacer les picots du transporteur suivant un trajet courbé qui croise le trajet de va-et-vient, de sorte qu'en fonctionnement, le brin porté par le guide-brin en mouvement, près de chaque extrémité de chaque course de va-et-vient, est bouclé autour d'un picot du transporteur à picots;
une surface (50) permet de supporter et de déplacer le substrat en feuille dans la direction longitudinale dans une position pour transférer le brin des picots vers le substrat en feuille, la position de transfert étant située entre les transporteurs à picots et au niveau d'une emprise formée par un moyen de retenue (52, 52') et la surface de support;
des moyens permettent de synchroniser les vitesses relatives du guide à mouvement de va-et-vient transversal et des transporteurs à picots pour assurer le bouclage du brin sur un picot du transporteur à chaque extrémité du trajet du brin, et
des moyens contrôlent le mouvement des moyens de retenue et de la surface de support à vitesses égales.

10. Appareil perfectionné suivant la revendication 9, dans lequel chaque transporteur à picots est un transporteur à picots rotatif, la surface de support est un tambour cylindrique rotatif, et les moyens de retenue comprennent une paire de courroies trapézoïdales.

11. Appareil perfectionné suivant la revendication 10, dans lequel la fente du guide-brin est une fente en forme de trou de serrure et chaque picot de chaque roue transporteuse à picots comporte un épaulement situé à une distance comprise entre 0,4 et 0,8 de celle mesurée de l'endroit où le picot est fixé à la roue jusqu'à l'extrémité supérieure du picot et séparant une partie de grand diamètre du picot d'une partie de plus petit diamètre du picot, la partie de plus petit diamètre étant la partie supérieure du picot.

12. Appareil perfectionné suivant la revendication 10 ou 11, dans lequel les roues transporteuses à picots sont inclinées de manière convergente ou divergente.
